# EUROPEAN PATENT SPECIFICATION

(11) **EP 0 579 488 B1**
(45) Date of publication and mention of the grant of the patent: **09.10.1996**
(21) Application number: 93305506.3
(22) Date of filing: 14.07.1993
(51) Int. Cl.: A61K 31/12

(54) **Composition for treating mastitis and metritis**
Zusammensetzung zur Behandlung von Mastitis und Metritis
Composition par le traitement de mastitis et métritis

(30) Priority: 14.07.1992 US 913034
(43) Date of publication of application: 19.01.1994
(73) Proprietor: Rajamannan, A.H.J., Minneapolis, Minnesota 55402 (US)
(72) Inventor: Rajamannan, A.H.J., Minneapolis, Minnesota 55402 (US)
(74) Representative: Pett, Christopher Phineas

(56) References cited:
- WO-A-92/00727
- US-A- 3 904 780
- Week 8413, Derwent Publications Ltd., London, GB; AN 84078370; & JP-A-59 029 618
- Week 8038, Derwent Publications Ltd., London, GB; AN 80-67467C
- PATENT ABSTRACTS OF JAPAN vol. 15, no. 500 (C-894), 1991; & JP-A-32 19 884

## Description

This invention relates to the treatment of mastitis and metritis, particularly but not exclusively, bovine mastitis and metritis.

Mastitis and metritis are two well known diseases which affect both humans and non-humans.

Although mastitis simply means inflammation of the mammary gland, it is clinically understood that the inflammation is caused by some infectious agent, usually bacteria. While there are various species of Staphylococcus organisms and Streptococcus organisms which cause mastitis in cows, cow-to-cow transmitted mastitis is usually caused by Staphylococcus aureus, Streptococcus agalactiae, and Streptococcus dysgalactiae. On the other hand, environmental mastitis, that is mastitis infection from the environment, is caused primarily by Streptococcus uberis and E. coli. Although there are a number of other bacterial organisms that cause bovine mastitis, the organisms referred to above are the most common causes of mastitis.

Mastitis continues to be a major cause of economic loss in the dairy industry even though there are methods of treating this disease. Currently, the primary method of treating mastitis in cows (inflammation of the udder) as well as treating metritis (inflammation of the uterus) is antibiotic therapy. However, the antibiotics used and their method of application tend to leave residues in the milk so that milk from cows treated with antibiotics must still be kept from human consumption.

This discarding of the milk from the cows under treatment is one of the more expensive aspects of mastitis. There are several reasons that milk contaminated with antibiotics should not be sold. Some people are allergic to antibiotics, especially penicillins, and fatalities have occurred relating to allergic reactions. If milk gained a public reputation for containing antibiotics, then sales of liquid milk might decline rapidly. Antibiotics consumed by humans at low dilute levels will also allow various pathogenic bacteria that are currently sensitive to these antibiotics to become resistant.

Further, antibiotics can destroy the bacterial cultures used in yoghurt and cheese manufacture. Finally, present regulations require milk from treated cows to be kept from the human food chain for three days. New regulations of longer holding times that are envisioned could deal the dairy industry a devastating blow.

There is therefore a need for a treatment of mastitis/metritis which overcomes some or substantially all of the problems outlined above. I have found that a combination of diacetyl and acetoin can be used to prepare a medicament for use in the treatment of mastitis and metritis.

Diacetyl is also known as 2,3-butanedione having the formula CH₃COCOCH₃. Acetoin is also known as 3-hydroxy 2-butanone, dimethyketol, or acetyl methylcarbinol having the formula CH₃CHOHCOCH₃.

In JP-A-5902 9618 there is described a dental caries inhibitor containing one or more of a group of over thirty possible compounds, including diacetyl and acetoin. Whilst technically this might allow for the possible combination of diacetyl and acetoin, no such combination is described. Diacetyl is also described as a possible active ingredient in an antiinflammatory ointment in SU-A-715087.

Accordingly I provide use of a composition comprising diacetyl and acetoin in the manufacture of a medicament for the treatment of mastitis and metritis.

This combination of diacetyl and acetoin, while having bactericidal characteristics, does not produce the problems associated with antibiotic therapy, namely residual antibiotics in the milk. A preferred feature of this invention is the use of the combination of diacetyl and acetoin in the treatment of bovine mastitis and bovine metritis.

Diacetyl and acetoin are also components of milk fermentation. Milk fermentation using bacteria such as Streptococcus diacetilactis, Streptococcus lactis, and Streptococcus cremoris produce compounds such as diacetyl, acetoin and volatile acids. These end products are of insufficient strength but they can be distilled or extracted and concentrated (what is known as "starter distillate"). Such concentrates have been used to add flavour to dairy foods.

In the present invention, the fermentation end products of diacetyl and acetoin may be concentrated by a factor of five (5 x starter distillate) or a factor of fifteen (15 x distillate) in order to acquire the required concentrations. The concentration of diacetyl in the present compositions may be in the range 15 parts per million (ppm) to 50,000 ppm. The concentration of acetoin may be in the range 2 to 20,000 ppm. Most preferably the concentrations of diacetyl and acetoin are about 7,500 ppm and 50 ppm respectively. Generally, the present invention includes the use of "concentrated" amount of diacetyl in comparison with a "trace" amount of acetoin.

With respect to bovine mastitis and metritis, the combination of diacetyl and acetoin may be infused into the udder cistern of the infected cow. Similarly, the diacetyl and acetoin may be infused into the uterus of an infected cow when treating metritis.

Compositions of diacetyl and acetoin may also include an anti-inflammatory agent such as cortisone or aloe vera juice. Antibiotics could also be added to the composition for enhancing the efficacy of the diacetyl and acetoin. Such antibiotics may include penicillin, synthetic penicillins (cloxacillin or ampicillin), antibiotic combinations (e.g. penicillin and streptomycin), tetracyclines or neomycins. Tea tree oil (Maleluca) may also be added to the composition to enhance anti-bacterial anti-inflammatory activity in the udder or uterus.

Experiments using the mixture of diacetyl and acetoin were performed on animals in test trials in the field. Mastitic cows usually have a swollen hot udder and show flaky milk on strip test.

The mastitis cows, when diagnosed with hot udder and showing flaky milk on strip test, were classified as to the type of bacterial infection such as E. coli, Streptococcus or Staphylococcus organisms.

### EXAMPLE I

Various concentrations of distilled diacetyl with traces of acetoin and volatile acids in the form of starter distillate were infused into the udder and observations on inflammation reduction, return to normalcy of milk, milk samples for positive bacterial presence, taste test and strip tests were performed.

The following results were obtained:

**TABLE I**

| Number of Cows | Type of Infection | Strength Diacetyl & Acetoin | Amount of Infusion | 100% Cure |
|---|---|---|---|---|
| 4 | E. Coli | 5 x distillate | 25cc per treat | 12 hrs |
| 4 | Streptococcus | 5 x distillate | 25cc per treat | 24-36hrs |
| 4 | Streptococcus | 15 x distillate | 25cc per treat | 12 hrs |
| 4 | Staphylococcus | 5 x distillate | 25cc per treat | 24-42hrs |
| 4 | Staphylococcus | 15 x distillate | 25cc per treat | 18hrs |

### EXAMPLE II

Cows with metritis were randomly divided into antibiotic groups and diacetyl groups.

Cows were infused with the traditional antibiotics or different doses of starter distillate diacetyl. Swabs were taken every 24 hours and the uterus was palpated for Signs of inflammation. The uteruses that did not respond to diacetyl were switched to antibiotics. Results were as follows:

**TABLE II**

| Number of Cows | Antibiotics | Time Return To Normal | Number of Cows | Diacetyl Acetoin | Return To Normal |
|---|---|---|---|---|---|
| 2 | Normal amounts | 24 hrs | 2 | 100cc (10x) | 1 in 48 hrs* |
| 2 | Normal amounts | 48 hrs | 2 | 100cc (10x) | 72 hrs |
| 2 | Normal amounts | 72 hrs | 2 | 100cc (10x) 3 times every 12 hours | 48 hrs |

| | | | | | |
|---|---|---|---|---|---|
| * One switched to antibiotics at end of 48 hours because of infection not cured | | | | | |

These trials show that diacetyl with acetoin can fully replace antibiotics to treat mastitis and most cases of metritis.

Since diacetyl and acetoin are approved for human consumption by the Food and Drug Administration of the united States of America for flavoring dairy products, the potential contamination of milk of diacetyl post treatment should not be a problem.

## Claims

1. Use of a composition comprising diacetyl and acetoin in the manufacture of a medicament for the treatment of mastitis.

2. Use of a composition comprising diacetyl and acetoin in the manufacture of a medicament for the treatment of metritis.

3. The use as claimed in Claim 1 or Claim 2 wherein the medicament includes an anti-inflammatory agent.

4. The use as claimed in Claim 3 wherein the anti-inflammatory agent is either cortisone or aloe vera juice.

5. The use as claimed in any preceding claim wherein the medicament includes Tea tree oil (Maleluca).

6. The use as claimed in any one of Claims 1, and 3 to 5 wherein the mastitis is bovine mastitis.

7. The use as claimed in any one of Claims 2 to 5 wherein the metritis is bovine metritis.

## Patentansprüche

1. Verwendung einer Zusammensetzung umfassend Diacetyl und Acetoin bei der Herstellung eines Arzneimittels zur Behandlung von Mastitis.

2. Verwendung einer Zusammensetzung umfassend Diacetyl und Acetoin bei der Herstellung eines Arzneimittels zur Behandlung von Metritis.

3. Verwendung wie in Anspruch 1 oder 2 beansprucht, worin das Arzneimittel ein anti-inflammatorisches bzw. entzündungshemmendes Mittel einschließt.

4. Verwendung wie in Anspruch 3 beansprucht, worin das anti-inflammatorische Mittel entweder Cortison oder Aloe vera-Saft ist.

5. Verwendung wie in einem der vorhergehenden Ansprüche beansprucht, worin das Arzneimittel Teebaumöl (Maleluca) einschließt.

6. Verwendung wie in einem der Ansprüche 1 und 3 bis 5 beansprucht, worin die Mastitis Rindermastitis ist.

7. Verwendung wie in einem der Ansprüche 2 bis 5 beansprucht, worin die Metritis Rindermetritis ist.

## Revendications

1. Utilisation d'une composition comprenant du diacétyle et de l'acétoïne dans la fabrication d'un médicament pour le traitement de la mastite.

2. Utilisation d'une composition comprenant du diacétyle et de l'acétoïne dans la fabrication d'un médicament pour le traitement de la métrite.

3. Utilisation selon la revendication 1 ou la revendication 2 dans laquelle le médicament inclut un agent anti-inflammatoire.

4. Utilisation selon la revendication 3 dans laquelle l'agent anti-inflammatoire est soit la cortisone soit le jus d'aloé véra.

5. Utilisation selon l'une quelconque des revendications précédentes dans laquelle le médicament inclut de l'huile d'arbre à Thé (Maléluca).

6. Utilisation selon l'une quelconque des revendications 1, et 3 à 5 dans laquelle la mastite est la mastite bovine.

7. Utilisation selon l'une quelconque des revendications 2 à 5 dans laquelle la métrite est la métrite bovine.
